# EUROPEAN PATENT APPLICATION

(11) **EP 2 163 547 A1**
(43) Date of publication of application: **17.03.2010**
(21) Application number: 08382035.7
(22) Date of filing: 16.09.2008
(51) Int. Cl.: C07D 263/20

(54) **Process for the preparation of an oxazolidinone antibacterial agent and intermediates thereof**

(71) Applicant: Unión Químico Farmacéutica, S.A. (UQUIFA), 08008 Barcelona (ES)
(72) Inventor: COMELY, Alexander Christian, 08004, BARCELONA (ES); XALMA ALONSO, Mónica, 08024, BARCELONA (ES); VERDAGUER ESPAULELLA, Xavier, 08500, VIC (ES); RAFECAS JANÉ, Llorenç, 43712, LLORENÇ DEL PENEDÉS (ES); SERRA MORTE, Sònia, 08028, BARCELONA (ES); LLORIS VIUDEZ, Maria, 08005, BARCELONA (ES); DOMINGO COTO, Antonio, 08029, BARCELONA (ES)
(74) Representative: Barlocci, Anna

(57) **Abstract**

Comprising a preparation process of linezolid from a compound of formula (IV) where R₁ is selected from a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group. Compound (IV) is submitted either first to an acetylation reaction and then to a dealkylation reaction or, alternatively, first to a dealkylation reaction and then to an acetylation reaction to yield linezolid. It also comprises new intermediate compounds useful in such a preparation process, which are obtained with high yields and high chemical and optical purity, and which are processed easily to linezolid.

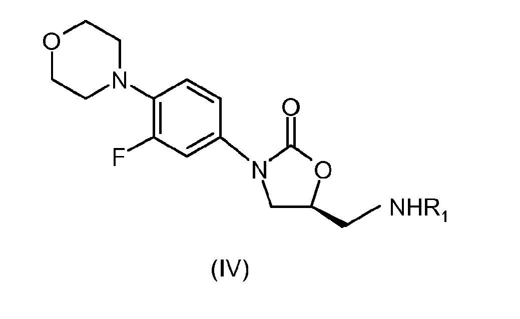

## Description

The present invention relates to a process for the preparation of linezolid, as well as to some new intermediates useful in such a preparation process.

### BACKGROUND ART

Linezolid is the International Non-proprietary Name (INN) of N-[[(5S)-3-[3-fluoro-4-(4-morpholinyl)phenyl]-2-oxo-5-oxazolidinyl]methyl]acetamide and CAS No. 165800-03-3, available under the brand name Zyvox®, Pfizer, in 600 mg tablets. Linezolid is currently used in treatment of bacterial infections, its main indications being nosocomial and community acquired pneumonia, skin and skin structure infections such as diabetic foot infection without concomitant osteomyelitis and, importantly, vancomycin resistant Enterococcus faecium infections.

The structure of Linezolid corresponds to formula (I):

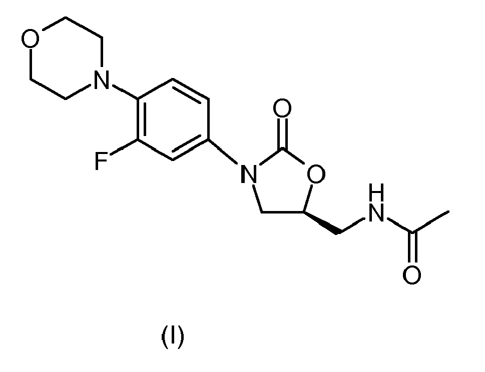

Different synthetic strategies for the preparation of Linezolid and its salts are known.

EP 717.738-A discloses certain oxazine and thiazineoxazolidinone derivatives, methods for their preparation, and pharmaceutical compositions using these compounds. Several preparation processes are reported in this document. They involve the hydroxyl activation of an alcohol precursor via formation of a mesylate which is then transformed into an amine. The transformation of the mesylate into the amine can be carried out through the formation of the corresponding azide followed by a reduction reaction or, alternatively, through the displacement of the sulfonate with potassium phthalimide followed by a deprotection reaction or, alternatively, by direct reaction with ammonium hydroxide. Finally, the last step is the acylation of the amine thus obtained.

According to Example 5 of the above patent application, Linezolid is prepared by transformation of (*R*)-N-[[3-(3-fluoro-4-morpholinyl]phenyl]-2-oxo-5-oxazolidinyl]methanol into its mesylate which involves treatment with methansulfonyl chloride, followed by treatment with sodium azide. The corresponding azide thus obtained is treated with hydrogen in the presence of 10% palladium/carbon to afford the corresponding amine, which is acetylated to yield linezolid. The main drawbacks of this method are the use of sodium azide, which is known to be an explosion hazard, and consequently the requirement of hydrogen and expensive palladium catalysts in reduction to the amine.

The other methods described in said European patent application to obtain oxazolidinones also have drawbacks. In EP 1.114.819, from the same applicant as EP 717.738-A, it is stated that the reaction of the sulfonate with aqueous ammonia needs high temperature and high pressure and, therefore, this process cannot be carried out in ordinary general purpose reactors and must be run in special reactors rated for high pressure. It is also stated that the reaction sequence which involves the formation of the sulfonate and its reaction with potassium phthalimide produces by-products which are difficult to separate from the desired product.

European patent application EP 1.114.819-A describes a process for the preparation of 5-aminomethyl substituted oxazolidinone amines by contacting some specific oxazolidinone sulfonates with ammonia at a pressure of less than about 30 psig.

Other processes for the preparation of linezolid are also known using a different synthetic approach based on the formation of the oxazolidinone ring from an arylcarbamate (cf. e.g. EP 1.028.940-A, EP 1.380.121-A and EP 1.328.509-A).

Although certain processes for the preparation of linezolid are known, there remains a need for new processes for the preparation of linezolid or its salts.

### SUMMARY OF THE INVENTION

The inventors have found a new preparation process of linezolid from new intermediate compounds, which proceeds with high yields and high chemical and optical purity. The process comprises the activation of an alcohol precursor of linezolid, amination with some specific amines bearing an alkyl group which may be removed, and the submission of the compound obtained to an acetylation reaction and to a dealkylation reaction, which last two steps can be carried out in either order. The process of the present invention is particularly advantageous in its practical industrial realization because it is much more cost effective, it has much less environmental impact and it is safer and is, therefore, much better suited for scale up than processes disclosed in EP 1.114.819.

For the transformation of the activated alcohol to the amine, the process of the present invention is advantageous since it avoids some of the drawbacks of previously described processes. When ammonia is used instead of the specific amines of the present invention, a very large excess of this reagent must be used which has an important environmental impact. The large volumes required reduce reactor product capacity and productivity. Yields are low, since over-alkylation reactions give rise to impurities difficult to purify (the secondary amine and the corresponding acetylated derivative). Furthermore, the process would be difficult to carry out at industrial scale. When phthalimide is used instead of the amines of the present invention, there are also difficulties. The reaction produces by-products which are difficult to separate from the desired product. The deprotection reaction of phthalimide compounds can be carried out with hydrazine or using basic conditions. The use of hydrazine involves several process difficulties and the basic conditions require high temperatures which may not be compatible with linezolid. Additionally, phthalimide salts are significantly more expensive than the amines used in the preparation process of the present invention.

Thus, an aspect of the present invention is to provide a process for the preparation of linezolid of formula (I), its pharmaceutically acceptable salts, or its solvates, including hydrates, 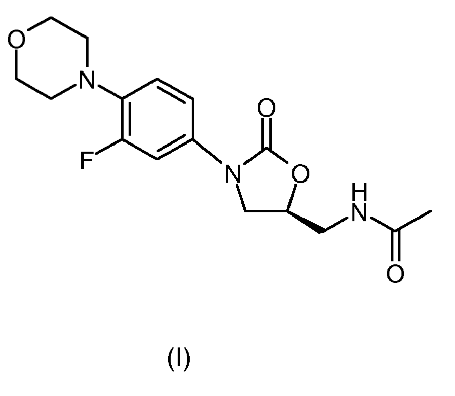 comprising submitting a compound of formula (IV) as defined below, first to an acetylation reaction and then to a dealkylation reaction, or, alternatively, first to a dealkylation reaction and then to an acetylation reaction; and, optionally, treating the compound (I) thus obtained with a pharmaceutically acceptable acid to form the corresponding pharmaceutically acceptable salt.

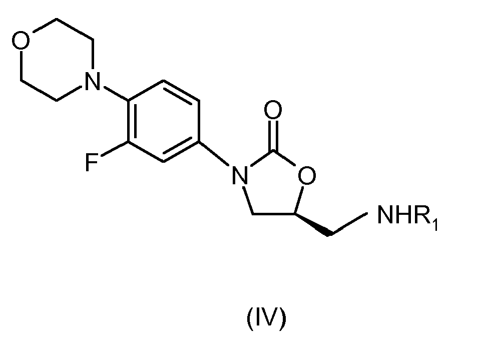

In compound (IV), R₁ is selected from a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group. These groups R₁ have in common that they may be removed from the N atom to which they are attached by a dealkylation reaction.

Another aspect of the present invention is to provide intermediate compounds which are useful in the preparation process described above. Thus, compounds of formula (IV) as defined above are new and are also part of the invention.

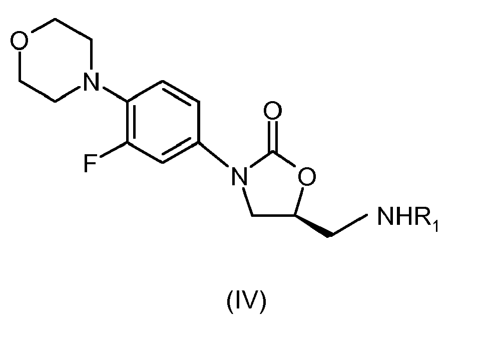

Compound (IV) is a key intermediate of the process which is obtained with high yields and high chemical and optical purity. The examples included in this document illustrate the results obtained regarding purity and yield of these intermediates. The compounds (IV) are crystalline and filter readily, and can be purified easily by conventional methods such as crystallization. Therefore, it is a significant contribution to the art to provide these intermediate compounds, which allow the preparation of linezolid by a simple process which proceeds with high yields and purity.

Compounds of formula (II), which are obtained when compounds of formula (IV) are first submitted to an acetylation reaction, are new and also form part of the invention.

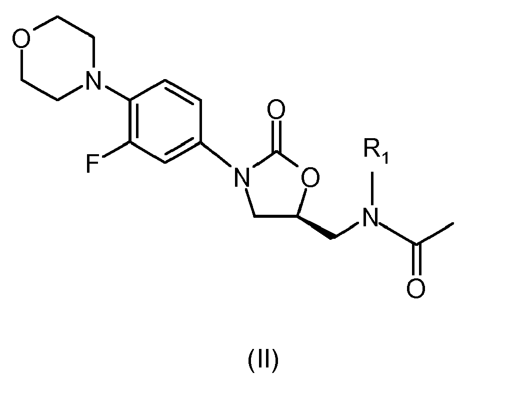

In formula (II), R₁ is selected from a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group.

### DETAILED DESCRIPTION OF THE INVENTION

As mentioned above the process of the present invention comprises submitting a compound (IV) to an acetylation reaction and to a dealkylation reaction, where these reactions can be carried out in any order.

In a preferred embodiment, the sequence of reactions to obtain linezolid (I) from a compound of formula (IV) is the following: first the acetylation reaction and then the dealkylation reaction.

The acetylation reaction of compound (IV) as defined above gives rise to the intermediate compound of formula (II) where R₁ is selected from a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group.

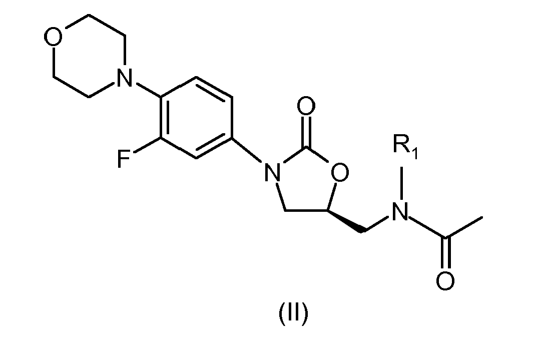

In a preferred embodiment, the acetylation reaction gives rise to compounds of formula (II) where R₁ is selected from allyl and tert-(C₄-C₈)alkyl.

In a more preferred embodiment, the acetylation reaction gives rise to compounds of formula (II) where R₁ is selected from allyl, tert-butyl, and tert-octyl.

The acetylation reaction of compound (IV) to yield compound (II) can be carried out using either an acetyl halide, such as acetyl chloride, or acetic anhydride in an appropriate solvent, and in the presence of a base. Appropriate solvents include chlorine containing solvents such as dichloromethane, ethers such as tert-butyl methyl ether, (C₃-C₇)-ketones such as methylisobutylketone, (C₆-C₈)-aromatic hydrocarbons such as toluene or xylene, or ethyl acetate. Suitable bases include pyridine or tertiary alkyl amines such as triethylamine. Generally, the reaction is carried out at a temperature comprised between room temperature and approximately 60 °C depending on the reagent used. If an acetyl halide is used, generally the reaction is carried out at room temperature. If acetic anhydride is used, then generally the reaction is carried out at a higher temperature.

The dealkylation reaction of the compound of formula (II), where R₁ is a straight or branched (C₃-C₁₀)-alkenyl radical attached to the N atom such that the C=C double bond is separated from the N atom by a methylene group, to yield linezolid (I) can be carried out with a mixture of palladium(II) chloride and sodium acetate in acetic acid. The dealkylation reaction of the compound of formula (II), where R₁ is a (C₄-C₁₀)-alkyl radical which is attached to the N atom by a tertiary C atom, can be carried out using a solution of hydrogen chloride or trifluoroacetic acid in an appropriate solvent. Example of appropriate solvents include chlorine containing solvents such as dichloromethane, ethers such as tert-butyl methyl ether, dioxane or tetrahydrofuran, (C₃-C₇)-ketones such as methylisobutylketone, (C₆-C₈)-aromatic hydrocarbons such as toluene or xylene, ethyl acetate, or water.

In another preferred embodiment, when compound (IV) is first submitted to a dealkylation reaction, an intermediate compound of formula (III) is obtained.

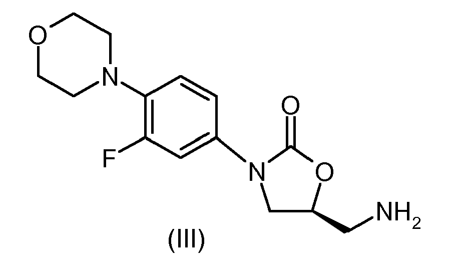

This dealkylation reaction of compound (IV), where R₁ is a straight or branched (C₃-C₁₀)-alkenyl radical attached to the N atom such that the C=C double bond is separated from the N atom by a methylene group, to yield compound (III) can be carried out using a mixture of palladium (II) acetate and triphenylphosphine in ethanol, or tetrakis(triphenylphosphine) palladium (0) (Pd(PPh₃)₄) in ethanol in the presence of dimethylbarbituric acid. The reaction can be carried out at a temperature comprised between room temperature and 60 °C approximately. The dealkylation reaction of the compound of formula (IV), where R₁ is a (C₄-C₁₀)-alkyl radical which is attached to the N atom by a tertiary C atom, can be carried out using a solution of hydrogen chloride or trifluoroacetic acid in an appropriate solvent. Example of appropriate solvents include chlorine containing solvents such as dichloromethane, ethers such as tert-butyl methyl ether, dioxane or tetrahydrofuran, (C₃-C₇)-ketones such as methylisobutylketone, (C₆-C₈)-aromatic hydrocarbons such as toluene or xylene, ethyl acetate, or water.

Compound (III) is then submitted to an acetylation reaction to give linezolid. This acetylation can be carried out using a mixture of acetic anhydride or acetyl chloride, triethylamine and an appropriate solvent. Example of appropriate solvents include chlorine containing solvents such as dichloromethane, ethers such as tert-butyl methyl ether or tetrahydrofuran, (C₃-C₇)-ketones such as methylisobutylketone, (C₆-C₈)-aromatic hydrocarbons such as toluene or xylene, or ethyl acetate. Preferably, the acetylation reaction is carried out using acetic anhydride in toluene as solvent and in the presence of triethylamine.

Linezolid obtained by the process of the present invention may be converted into pharmaceutically acceptable salts by known methods described in the art, for instance, by the reaction of linezolid free base with a sufficient amount of a pharmaceutically acceptable acid to yield the corresponding salt.

The term "pharmaceutically acceptable salts" used herein encompasses any salt formed from organic and inorganic acids. Examples of inorganic acids include hydrochloric acid, hydrobromic acid, hydroiodic acid, nitric acid, perchloric acid, sulfuric acid or phosphoric acid. Examples of organic acids include methansulfonic acid, trifluoromethansulfonic acid, ethanesulfonic acid, 2-hydroxyethanesulfonic acid, benzenesulfonic acid, p-toluensulfonic acid, fumaric acid, citric acid, oxalic acid, acetic acid and malic acid. There is no limitation regarding the salts, except that if used for therapeutic purposes, they must be pharmaceutically acceptable.

The intermediate compound of formula (IV) as defined above can be obtained by submitting a compound of formula (V), where R₂ is a radical selected from the group consisting of (C₁-C₄)-alkyl, phenyl, and phenyl mono- or disubstituted by a (C₁-C₄)-alkyl radical to an amination reaction. The amination reaction is carried out by reacting said compound (V) with an amine of formula R₁NH₂ where R₁ is selected from a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group. 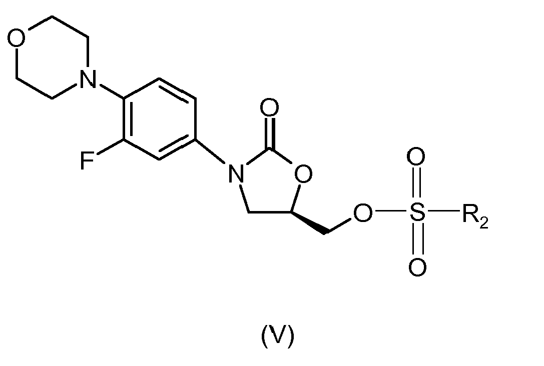 Preferred sulfonate compounds of formula (V) for use in the process of the present invention are those where the sulfonate (V) is a mesylate (R₂ = methyl), a besylate (R₂ = phenyl) or a tosylate (R₂ = 4-methylphenyl). The most preferred sulfonate (V) is the mesylate.

In a preferred embodiment, the amine used in the amination reaction is an allyl or a tert-(C₄-C₈)alkyl amine. In a more preferred embodiment, the amine used is allylamine, tert-butylamine or tert-octylamine.

In another preferred embodiment, the amination reaction is carried out using a (C₁-C₆)-alcohol as solvent. More preferably, the alcohol used is isopropanol.

In another preferred embodiment, the sulfonate of formula (V) can be prepared from the corresponding alcohol of formula (VI) by reaction with the corresponding sulfonyl chloride of formula Cl-SO₂-R₂, where R₂ has the same meaning defined above for compound (V). 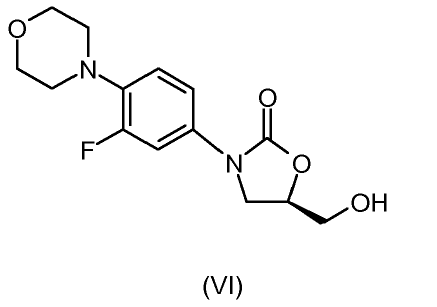 This reaction can be carried out in an appropriate solvent and in the presence of a tertiary amine, at a temperature comprised between 0 °C and 70 °C, preferably at a temperature comprised between 0 °C and room temperature. More preferably, the reaction is carried out at low temperatures. Common solvents for this reaction include chlorine-containing solvents such as methylene chloride or 1,2-dichloroethane, aromatic hydrocarbons such as toluene or xylene, ethers such as tetrahydrofuran, (C₃-C₇)-ketones such as methylethylketone, and dimethylformamide. Examples of suitable tertiary amines are diisopropylethylamine and triethylamine.

The starting alcohol of formula (VI) is commercial and can be prepared by any of the methods known in the art.

Furthermore, the present invention covers all possible combinations of particular and preferred embodiments described hereinabove.

Throughout the description and claims the word "comprise" and variations of the word, are not intended to exclude other technical features, additives, components, or steps.

Additional objects, advantages and features of the invention will become apparent to those skilled in the art upon examination of the description or may be learned by practice of the invention. The following examples and drawings are provided by way of illustration, and they are not intended to be limiting of the present invention.

### EXAMPLES

### Example 1: Preparation of (R)-3-(3-fluoro-4-morpholinophenyl)-2-oxazolidin-5-yl)methyl methanesulfonate (compound (V) with R₂ = methyl)

To a solution of ((*R*)-3-(3-fluoro-4-morpholinophenyl)-2-oxo-5-oxazolidinyl)methanol (20 g, 67.5 mmol) in tetrahydrofuran (220 mL, 11 mL/g) at room temperature under inert atmosphere was added triethylamine (12.2 mL, 87.8 mmol, 1.3 eq). The mixture was cooled in an ice bath and methanesulfonyl chloride (6.8 mL, 87.8 mmol, 1.3 eq) was added dropwise over approximately 15 min (resulting in an increase in Tᵢₙₜ from 8 to 15 °C. A pale lilac precipitate forms immediately). The ice bath is removed and the suspension is stirred for 3.5 h. The suspension was filtered at room temperature and the solids (32.9 g) were suspended in water (165 mL, 5 vol) and stirred for 2 h at room temperature. The suspension was filtered and a second suspension in water was carried out (as above). The suspension was filtered and the collected solids were dried in-vacuo (60 °C, 2-4 mm Hg) to give ((R)-3-(3-fluoro-4-morpholinophenyl)-2-oxazolidin-5-yl)methyl methanesulfonate (24.4 g, 96%) as a pale lilac powder.
¹H NMR (400 MHz, acetone-d₆): δ 7.57 (dd, 1H, ArH, *J* 2.8 and 15 Hz), 7.23 (ddd, 1H, ArH, *J* 2.7 and 9 Hz), 7.07 (t, 1H, ArH, *J* 9Hz), 5.05-5.01 (m, 1H, CH), 4.58 (qd, 2H, CH₂, *J* 3, 5, 8.5 and 12 Hz), 4.30 (t, 1H, CH, *J* 9 Hz), 3.99 (dd, 1H, CH, *J* 6 and 9 Hz), 3.79-3.77 (m, 4H, CH₂), 3.20 (s, 3H, CH₃), 3.03-3.01 (m, 4H, CH₂) ppm.
Mp 178.7 - 180.6 °C
[α]_{D}-55.5° (c 0.98, acetone)

### Example 2: Preparation of (S)-5-((allylamino)methyl)-3-(3-fluoro-4-morpholinophenyl)oxazolidin-2-one (compound (IV) with R₁ = allyl)

To a suspension of ((*R*)-3-(3-fluoro-4-morpholinophenyl)-2-oxazolidin-5-yl)methyl methanesulfonate (2 g, 5.34 mmol) in isopropanol (5 mL, 2.5 vol) in a thick-walled tube with Teflon screw cap was added allylamine (1.0 mL, 13.36 mmol, 2.5 eq). The tube was sealed and the mixture was heated in an oil bath at 120 °C for 4 h. The mixture was cooled to room temperature, was concentrated to dryness and was redissolved in toluene (10 mL) and water (4 mL). The mixture was acidified to pH 1 with 6 M hydrochloric acid, the aqueous phase was separated and the organic phase was extracted with water (4 mL). The combined aqueous layers were washed with toluene (4 mL) and were basified to pH 10 with 6 M aqueous sodium hydroxide solution. Extraction with toluene (4 x 4 mL) at 50 °C, drying over magnesium sulfate and concentration gave (S)-5-((allylamino)methyl)-3-(3-fluoro-4-morpholinophenyl)oxazolidin-2-one (1.68 g, 94%, HPLC 94%, 99.9% ee) as a yellow oil.
¹H NMR (400 MHz, CDCl₃): δ 7.45 (dd, 1H, ArH, *J* 2.8 and 14.4 Hz), 7.14 (ddd, 1H, ArH, *J* 1.7, 2.4 and 8.7 Hz), 6.93 (t, 1H, ArH, *J* 9 Hz), 5.91-5.81 (m, 1H, =CH), 5.22-5.11 (m, 2H, =CH₂), 4.79-4.72 (m, 1H, CH), 4.00 (t, 1H, *J* 8.8 Hz, CH), 3.88-3.86 (m, 4H, CH₂), 3.84-3.81 (m, 1H, CH), 3.32-3.30 (m, 2H,
CH₂), 3.06-3.04 (m, 4H, CH₂), 2.94 (qd, 2H, *J* 4.4, 5.6 and 13 Hz) ppm. [α]_{D} -57.3° (c 1.04, CHCl₃)

### Example 3: Preparation of (S)-5-((tert-butylamino)methyl)-3-(3-fluoro-4-morpholinophenyl)oxazolidin-2-one (compound (IV) with R₁ = tert-butyl)

To a suspension of ((*R*)-3-(3-fluoro-4-morpholinophenyl)-2-oxazolidin-5-yl)methyl methanesulfonate (19 g, 50.7 mmol) in isopropanol (47.5 mL, 2.5 vol) in a sealed reactor was added tert-butylamine (13.3 mL, 126 mmol, 2.5 eq) and the mixture was heated to Tᵢₙₜ 120 °C. After 3 d, the reaction is cooled to room temperature whereupon precipitation is observed. The suspension is then stirred for 1 h in an ice bath. Filtration, washing with isopropanol (2 x 19 mL, 1 vol) and water (2 x 19 mL, 1 vol) and drying gave (S)-5-((tertbutylamino)methyl)-3-(3-fluoro-4-morpholinophenyl)oxazolidin-2-one (15.3 g, 89%, HPLC 97.1%, 99.9% ee) as a white powder.
¹H NMR (400 MHz, CDCl₃): δ 7.45 (dd, 1H, ArH, *J* 2.8 and 14 Hz), 7.14 (dd, 1H, ArH, *J* 2 and 8 Hz), 6.93 (t, 1H, ArH, *J* 9Hz), 4.74-4.60 (m, 1H, CH), 3.99 (t, 1H, CH, *J* 9 Hz), 3.88-3.85 (m, 5H, CH₂), 3.06-3.04 (m, 4H, CH₂), 2.88 (qd, 2H, CH₂, *J* 5, 6, 9 and 12 Hz), 1.1 (s, 9H, CH₃) ppm.
Mp 142.2 - 143.3 °C
[α]_{D}-52.4 (c 1.01, CHCl₃)

### Example 4: Preparation of (S)-5-((tert-octylamino)methyl)-3-(3-fluoro-4-morpholinophenyl)oxazolidin-2-one (compound (IV) with R₁ = tert-octyl)

To a suspension of ((*R*)-3-(3-fluoro-4-morpholinophenyl)-2-oxazolidin-5-yl)methyl methanesulfonate (0.5 g, 1.33 mmol) in cyclohexanol (7 mL, 14 vol) was added tert-octylamine (1.1 mL, 6.65 mmol, 5 eq). The mixture was heated in an oil bath at 150 °C for 5 h and was cooled to room temperature. The mixture was dissolved in ethyl acetate (10 mL) and water (5 mL) and was acidified to pH 1 with 1 M hydrochloric acid. The aqueous phase was decanted and the organic phase was extracted with water (2 x 5 mL). The combined aqueous phases were washed with ethyl acetate (5 mL) and were basified to pH 10 with 1 M sodium hydroxide solution. Extraction with dichloromethane (4 x 5 mL), drying over magnesium sulfate and concentration gave (*S*)-5-((tert-octylamino)methyl)-3-(3-fluoro-4-morpholinophenyl)oxazolidin-2-one (380 mg, 70%, HPLC 92.8%, 99.9% ee) as a white solid.
¹H NMR (400 MHz, CDCl₃): δ 7.44 (dd, 1H, ArH, *J* 2.8 and 14 Hz), 7.14 (dd, 1H, ArH, *J* 2 and 8 Hz), 6.93 (t, 1H, ArH, *J* 9Hz), 4.73-4.60 (m, 1H, CH), 3.98 (t, 1H, CH, *J* 9 Hz), 3.89-3.85 (m, 5H, CH₂), 3.06-3.04 (m, 4H, CH₂), 2.88 (qd, 2H, CH₂, *J* 4, 5, 12 and 43 Hz), 1.40 (s, 2H, CH₂), 1.14 (s, 6H, CH₃), 0.99 (s, 9H, CH₃) ppm.
Mp 123.5 - 124.8 °C
[α]_{D} -53.7 (c 1.07, CHCl₃)

### Example 5: Preparation of (S)-5-((tert-octylamino)methyl)-3-(3-fluoro-4-morpholinophenyl)oxazolidin-2-one (compound (IV) with R₁ = tert-octyl)

A suspension of ((*R*)-3-(3-fluoro-4-morpholinophenyl)-2-oxazolidin-5-yl)methyl methanesulfonate (2.0 g, 5.34 mmol), isopropanol (5 mL, 2.5 vol) and tert-octylamine (2.2 mL, 13.36 mmol, 2.5 eq) was heated at 120 °C for 22 h in a sealed reactor. The temperature was increased to 150 °C for a further 19 h. The mixture was cooled to 0 °C and was stirred for 1 h. The resulting suspension was filtered, the solids were washed with cold isopropanol (4 mL, 2 vol) and with cold water (2 x 4 mL). Drying in vacuo gave (S)-5-((tertoctylamino)methyl)-3-(3-fluoro-4-morpholinophenyl)oxazolidin-2-one (1.70 g, 78%, HPLC 96.6%) as a white solid. For characterization, see Example 4.

### Example 6: Preparation of N-allyl-N-(((S)-3-(3-fluoro-4-morpholinophenyl)-2-oxazolidin-5-yl)methyl)acetamide (compound (II) with R₁ = allyl)

To a solution of (*S*)-5-((allylamino)methyl)-3-(3-fluoro-4-morpholinophenyl)oxazolidin-2-one (257 mg, 0.77 mmol) in dichloromethane (17 mL) under inert atmosphere was added triethylamine (218 µl, 1.57 mmol, 2.0 eq) and acetyl chloride (111 µl, 1.57 mmol, 2.0 eq) and the mixture was stirred at room temperature for 4.5 h. The mixture was washed with water (3 x 5 mL) and the combined aqueous layers were extracted with dichloromethane (2 x 5 mL). The combined organic layers were dried over magnesium sulfate and were concentrated to dryness in vacuo to give *N*-allyl-N-(((S)-3-(3-fluoro-4-morpholinophenyl)-2-oxazolidin-5-yl)methyl)acetamide (239 mg, 82%, HPLC 97.5%) as a oil.
¹H NMR (400 MHz, CDCl₃): δ 7.44 (dd, 1H, ArH, *J* 2 and 14.4 Hz), 7.09 (ddd, 1H, ArH, *J* 1.2 and 8.8 Hz), 6.92 (t, 1H, ArH, *J* 9 Hz), 5.85-5.75 (m, 1H, =CH), 5.22 (d, 1H, =CHH, *J* 10 Hz), 5.15 (d, 1H, =CHH, *J* 18 Hz), 4.90-4.82 (m, 1H, CH), 4.17-4.08 (m, 2H, CH₂), 4.02 (t, 1H, CH, *J* 8 Hz), 3.92-3.88 (m, 1H, CH), 3.88-3.85 (m, 4H, CH₂), 3.75 (dd, 1H, CH, *J* 7 and 9 Hz), 3.47 (dd, 1H, CH, *J* 7 and 14 Hz), 3.06-3.04 (m, 4H, CH₂), 2.12 (s, 3H, CH₃) ppm.
[α]_{D} -14.9 (c 1.01, CHCl₃)

### Example 7: Preparation of N-tert-butyl-N-(((S)-3-(3-fluoro-4-morpholinoiphenyl)-2-oxazolidin-5-yl)methyl)acetamide (compound (II) with R₁ = tert-butyl)

To a suspension of (*S*)-5-((tert-butylamino)methyl)-3-(3-fluoro-4-morpholinophenyl)oxazolidin-2-one (1 g, 2.84 mmol) in ethyl acetate (2 mL, 2 vol) was added triethylamine (0.47 mL, 3.4 mmol, 1.2 eq) and acetic anhydride (0.32 mL, 3.4 mmol, 1.2 eq) and the mixture was heated to 55 °C for 18 h. The mixture was cooled to room temperature, was stirred at this temperature for 1 h and then at 0 °C for 1 h. Filtration, washing with cold ethyl acetate (2 x 1 mL, 1 vol) and drying in vacuo gave *N*-tert-butyl-*N*-(((S)-3-(3-fluoro-4-morpholinophenyl)-2-oxazolidin-5-yl)methyl)acetamide (0.94 g, 84%, HPLC 99.2%, 99.9% ee) as a beige solid.
¹H NMR (400 MHz, CDCl₃): δ 7.44 (dd, 1H, ArH, *J* 2.8 and 14 Hz), 7.11 (ddd, 1H, ArH, *J* 1.2, 1, 2.4 and 9 Hz), 6.93 (t, 1H, ArH, *J* 9Hz), 4.78-4.72 (m, 1H, CH), 4.09 (t, 1H, CH, *J* 9 Hz), 3.88-3.86 (m, 5H, CHH + CH₂), 3.74-3.63 (m, 3H, CH, CH₂), 3.07-3.04 (m, 4H, CH₂), 2.23 (s, 3H, CH₃), 1.51 (s, 9H, CH₃)
ppm.
Mp 185.5 - 186.9 °C
[α]_{D} -54.2 ° (c 1.03, CHCl₃)

### Example 8: Preparation of N-tert-butyl-N-(((S)-3-(3-fluoro-4-morpholinophenyl)-2-oxazolidin-5-yl)methyl)acetamide (compound (II) with R₁= tert-butyl)

To a solution of (*S*)-5-((tert-butylamino)methyl)-3-(3-fluoro-4-morpholinophenyl)oxazolidin-2-one (177 mg, 0.50 mmol) in dichloromethane (3.5 mL) under inert atmosphere was added triethylamine (84 µl, 0.60 mmol, 1.2 eq) and acetyl chloride (43 µl, 0.60 mmol, 1.2 eq) and the mixture was stirred at room temperature for 4 h. The mixture was washed with water (4 x 5 mL) and the combined aqueous layers were extracted with dichloromethane (2 x 10 mL). The combined organic layers were dried over magnesium sulfate and were concentrated to dryness in vacuo to give *N*-tert-butyl-*N*-(((S)-3-(3-fluoro-4-morpholinophenyl)-2-oxazolidin-5-yl)methyl)acetamide (161 mg, 81 %) as a beige solid.
For characterization, see Example 7.

### Example 9: Preparation of N-tert-octyl-N-(((S)-3-(3-fluoro-4-morpholinophenyl)-2-oxazolidin-5-yl)methyl)acetamide (compound (II) with R₁= tert-octyl)

To a solution (*S*)-5-((tert-octylamino)methyl)-3-(3-fluoro-4-morpholinophenyl) oxazolidin-2-one (200 mg, 0.49 mmol) in dichloromethane (1 mL) under inert atmosphere was added triethylamine (82 µl, 0.59 mmol, 1.2 eq) and acetyl chloride (42 µl, 0.59 mmol, 1.2 eq) and the mixture was stirred at room temperature for 7 h. The mixture was diluted with dichloromethane, was washed with water (3 x 1 mL) and the combined aqueous layers were extracted with dichloromethane (1 x 1 mL). The combined organic layers were dried over magnesium sulfate and were concentrated to dryness in vacuo to give an oil which was purified by flash chromatography (silica, dichloromethane/ethyl acetate 0.5-20%) to give *N*-tert-octyl-*N*-(((S)-3-(3-fluoro-4-morpholinophenyl)-2-oxazolidin-5-yl)methyl)acetamide (139 mg, 63%, HPLC 96.5%) as a white solid.
¹H NMR (400 MHz, CDCl₃): δ 7.43 (dd, 1H, ArH, *J* 2 and 15 Hz), 7.11 (dd, 1H, ArH, *J* 2 and 8 Hz), 6.93 (t, 1H, ArH, *J* 9Hz), 4.80-4.70 (m, 1H, CH), 4.11 (t, 1H, CH, *J* 9 Hz), 3.88-3.85 (m, 4H, CH₂), 3.78-3.64 (m, 3H), 3.07-3.02 (m, 4H, CH₂), 2.21 (s, 3H, CH₃), 1.97 (b, 2H, CH₂), 1.56 (s, 3H, CH₃), 1.54 (s, 3H, CH₃), 0.97 (s, 9H, CH₃) ppm.
Mp 146.7-147.9 °C
[α]_{D} -33.9 (c 1.0, CHCl₃)

### Example 10: Preparation of linezolid by dealkylation of N-allyl-N-(((S)-3-(3-fluoro-4-moripholinoiphenyl)-2-oxazolidin-5-yl)methyl)acetamide using palladium(II) chloride, sodium acetate, acetic acid

To a solution of *N*-allyl-*N*-(((*S*)-3-(3-fluoro-4-morpholinophenyl)-2-oxazolidin-5-yl)methyl)acetamide (239 mg, 0.63 mmol) in acetic acid (4 mL) and water (1 mL) was added palladium(II) chloride (112 mg, 0.63 mmol, 1 eq) and sodium acetate (104 mg, 1.27 mmol, 2 eq) and the mixture was stirred at room temperature for 5 d. The suspension was filtered through celite, the resulting phases were decanted and the aqueous layer was extracted with dichloromethane (3 x 5 mL). The combined dichloromethane layers were dried over magnesium sulfate, were concentrated in vacuo and were purified by column chromatography (silica, dichloromethane/methanol 0-3%) to give *N*-(((S)-3-(3-fluoro-4-morpholinophenyl)-2-oxazolidin-5-yl)methyl)acetamide, linezolid (30 mg, 14%) as an oil.
¹H NMR (400 MHz, CDCl₃): δ 7.43 (dd, 1H, ArH, *J* 2 and 14 Hz), 7.07 (db, 1H, ArH), 6.92 (t, 1H, ArH, *J* 9Hz), 6.33 (b, 1H, NH), 4.80-4.75 (m, 1H, CH), 4.02 (t, 1H, CH, *J* 9 Hz), 3.88-3.86 (m, 4H, CH₂), 3.77-3.60 (m, 3H, CH, CH₂), 3.06-3.04 (m, 4H, CH₂), 2.02 (s, 3H, CH₃) ppm.
Mp 177.5 - 178.1 °C
[α]_{D} -13.0 (c 0.99, CHCl₃)

### Example 11: Preparation of linezolid by dealkylation of N-tert-butyl-N-(((S)-3-(3-fluoro-4-morpholinophenyl)-2-oxazolidin-5-yl)methyl)acetamide using hydrogen chloride/dioxane

To a suspension of *N*-tert-butyl-*N*-(((*S*)-3-(3-fluoro-4-morpholinophenyl)-2-oxazolidin-5-yl)methyl)acetamide (496 mg, 1.26 mmol) in dioxane (1 mL) was added 4 M hydrogen chloride in dioxane (630 µL, 2.52 mmol, 2 eq) and the mixture was heated to 70 °C for 7 h. On cooling to room temperature, water (0.5 mL) was added and the mixture was basified with 2 M aqueous sodium hydroxide to pH 9. The resulting solution was concentrated to dryness and the resulting residue was resuspended in water (0.5 mL), was filtered and was washed with water (2 x 0.5 mL) to give *N*-(((S)-3-(3-fluoro-4-morpholinophenyl)-2-oxazolidin-5-yl)methyl)acetamide, linezolid (330 mg, 78%, HPLC 99.1%, 99.9% ee) as an off-white solid.
¹H NMR (400 MHz, CDCl₃): δ 7.43 (dd, 1H, ArH, *J* 2 and 14 Hz), 7.07 (db, 1H, ArH), 6.92 (t, 1H, ArH, *J* 9Hz), 6.33 (b, 1H, NH), 4.80-4.75 (m, 1H, CH), 4.02 (t, 1H, CH, *J* 9 Hz), 3.88-3.86 (m, 4H, CH₂), 3.77-3.60 (m, 3H, CH, CH₂), 3.06-3.04 (m, 4H, CH₂), 2.02 (s, 3H, CH₃) ppm.
Mp 177.5 - 178.3 °C
[α]_{D}-11.1° (c 1.02, CHCl₃)

### Example 12: Preparation of linezolid by dealkylation of N-tert-butyl-N-(((S)-3-(3-fluoro-4-morpholinophenyl)-2-oxazolidin-5-yl)methyl)acetamide using aqueous hydrochloric acid / ethyl acetate

To a solution of *N*-tert-butyl-*N*-(((*S*)-3-(3-fluoro-4-morpholinophenyl)-2-oxazolidin-5-yl)methyl)acetamide (1 g, 2.3 mmol) in ethyl acetate (5 mL) was added 6 M hydrochloric acid aq (850 µL, 5.1 mmol, 2 eq) and the solution was stirred at 55 °C for 3.5 h. 6 M sodium hydroxide solution was added to pH 10 and the ethyl acetate was distilled. The resulting suspension was stirred at 0 °C for 1 h and was filtered. The collected solids were washed with water (2 x 1 mL) and dried in vacuo to give *N*-(((*S*)-3-(3-fluoro-4-morpholinophenyl)-2-oxazolidin-5-yl)methyl)acetamide, linezolid (645 mg, 75%, HPLC 99%, 99.9% ee) as an off-white solid. For characterization, see Example 11.

### Example 13: Preparation of linezolid by deprotection of N-tert-octyl-N-(((S)-3-(3-fluoro-4-morpholinophenyl)-2-oxazolidin-5-yl)methyl)acetamide using aqueous hydrochloric acid / ethyl acetate

To a suspension of *N*-tert-octyl-*N*-(((*S*)-3-(3-fluoro-4-morpholinophenyl)-2-oxazolidin-5-yl)methyl)acetamide (160 mg, 0.36 mmol) in ethyl acetate (0.8 mL, 5 vol) was added hydrochloric acid (6 M, 120 µL, 0.712 mmol) and the mixture was heated at 55 °C for 2 h._6 M aqueous sodium hydroxide solution was added to pH 10 and the ethyl acetate was distilled. The resulting suspension was stirred at 0 °C for 1 h and was filtered. The collected solids were washed with water (2 x 0.2 mL) and dried in vacuo to give *N*-(((S)-3-(3-fluoro-4-morpholinophenyl)-2-oxazolidin-5-yl)methyl)acetamide, linezolid (104 mg, 87%, HPLC 86.6%) as an off-white solid. For characterization, see Example 11.

### Example 14: Preparation of (S)-5-(aminomethyl)-3-(3-fluoro-4-morpholinophenyl)oxazolidin-2-one (compound (III))

To a solution of palladium(II) acetate (1 mg, 1.5 µg, 0.01 eq) and triphenylphosphine (1.5 mg, 5.9 µg, 0.04 eq) in ethanol (1 mL) under argon was added a solution of (*S*)-5-((allylamino)methyl)-3-(3-fluoro-4-morpholinophenyl)oxazolidin-2-one (0.5 g, 1.49 mmol) in ethanol (0.6 mL) by canula under argon. The second solution was washed through with further ethanol (0.5 mL) and 1,3-dimethylbarbituric acid (279 mg, 1.79 mmol, 1.2 eq) was added. The mixture was heated to 35 °C for 5 h and the resulting solution was cooled and concentrated to dryness. The residue was redissolved in dichloromethane (4 mL) and water (4 mL) and the mixture was treated with 1 M hydrochloric acid to pH 1. The organic layer was decanted, was extracted with water (5 mL) and the combined aqueous layers were washed with dichloromethane (2 x 5 mL). The aqueous phase was basified with 4 M aqueous sodium hydroxide solution to pH 10 and was extracted with dichloromethane (4 x 8 mL). The combined organic layers were dried over magnesium sulfate and were concentrated to dryness to give (S)-5-(aminomethyl)-3-(3-fluoro-4-morpholinophenyl)oxazolidin-2-one (355 mg, 81 %, HPLC 93.2%, 99.9% ee) as a beige solid.
¹H NMR (400 MHz, CDCl₃): δ 7.46 (dd, 1H, ArH, *J* 2.4 and 14 Hz), 7.14 (ddd, 1H, ArH, *J* 1.2, 2 and 9 Hz), 6.93 (t, 1H, ArH, *J* 9Hz), 4.70-4.63 (m, 1H, CH), 4.01 (t, 1H, CH, *J* 9 Hz), 3.88-3.86 (m, 4H, CH₂), 3.82 (dd, 1H, CH, *J* 6, 7 and 8 Hz), 3.13-2.95 (m, 6H, CH₂), 1.4-1.3 (b, 2H, NH₂) ppm.
Mp 95.0 - 97.7 °C
[α]_{D} -47.9 (c 1.02, CHCl₃)

### Example 15: Preparation of linezolid by acetylation of (S)-5-(aminomethyl)-3-(3-fluoro-4-morpholinophenyl)oxazolidin-2-one using Ac₂O, NEt₃, toluene

To a suspension of (*S*)-5-(aminomethyl)-3-(3-fluoro-4-morpholinophenyl)oxazolidin-2-one (III) (452 mg, 1.53 mmol) in toluene (2.3 mL) was added triethylamine (215 µL, 1.54 mmol, 1 eq) and acetic anhydride (146 µL, 1.54 mL, 1 eq) and the mixture was stirred at room temperature for 5.5 h. The suspension was cooled in an ice bath for 30 min, was filtered and the collected solids were washed with cold toluene (2 x 0.5 mL) and were dried in vacuo to give *N*-((*(*S)-3-(3-fluoro-4-morpholinophenyl)-2-oxazolidin-5-yl)methyl)acetamide, linezolid (390 mg, 77%, HPLC 99.1%, 99.9% ee) as an off-white solid. For characterization, see Example 11.

## Claims

1. A process for the preparation of linezolid of formula (I), or a pharmaceutically acceptable salt thereof, or a solvate thereof, including a hydrate, 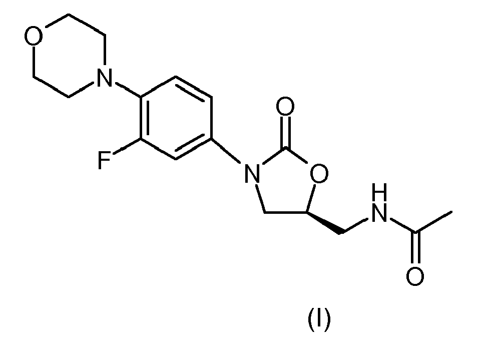 comprising submitting a compound of formula (IV) 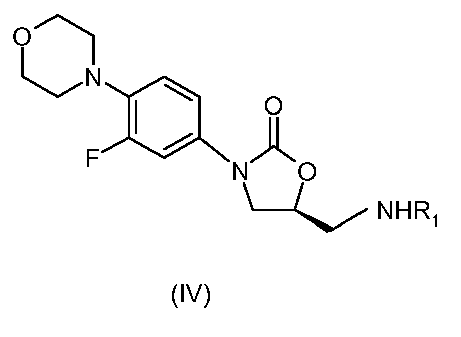 wherein R₁ is selected from the group consisting of a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group;
either first to an acetylation reaction and then to a dealkylation reaction or, alternatively, first to a dealkylation reaction and then to an acetylation reaction; and, optionally, treating the compound (I) thus obtained with a pharmaceutical acceptable acid to form the corresponding salt.

2. The process according to claim 1, wherein the R₁ is selected from allyl and tert-(C₄-C₈)alkyl.

3. The process according to claim 2, wherein R₁ is selected from the group consisting of allyl, tert-butyl, and tert-octyl.

4. The process according to any of the claims 1-3, wherein the compound of formula (IV) first is submitted to the acetylation reaction giving a compound of formula (II), 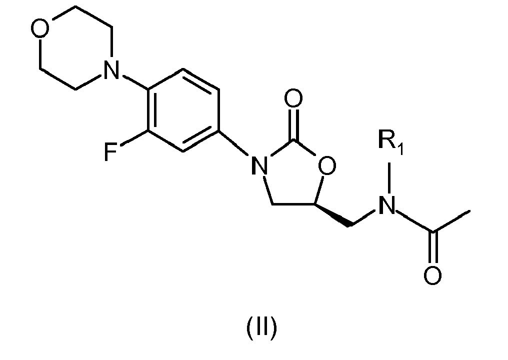 wherein R₁ has the same meaning as in the compound of formula (IV), and then to the dealkylation reaction.

5. The process according to any of the claims 1-4, wherein a compound of formula (V), 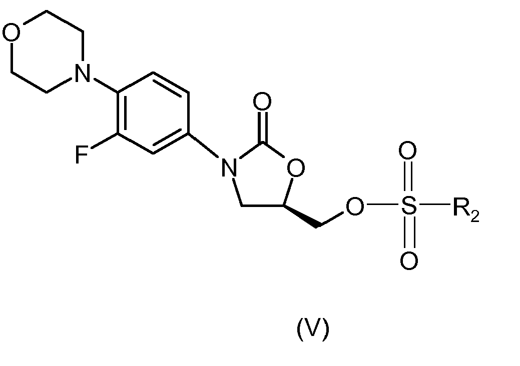 wherein R₂ is a radical selected from the group consisting of (C₁-C₄)-alkyl, phenyl, and phenyl mono- or disubstituted by a (C₁-C₄)-alkyl radical,
is submitted to an amination reaction with an amine of formula R₁NH₂ where R₁ is selected from the group consisting of a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group, to give the compound of formula (IV) as defined in claim 1.

6. A process for the preparation of a compound of formula (IV) 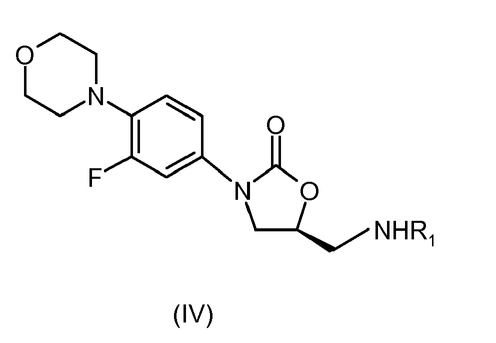 wherein R₁ is as defined in claim 1;
comprising submitting a compound of formula (V), 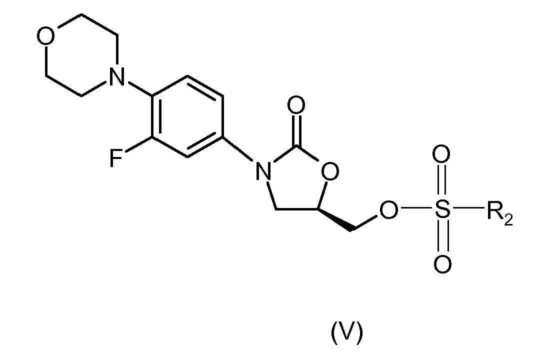 wherein R₂ is a radical selected from the group consisting of (C₁-C₄)-alkyl, phenyl, and phenyl mono- or disubstituted by a (C₁-C₄)-alkyl radical,
to an amination reaction with an amine of formula R₁NH₂ where R₁ is selected from the group consisting of a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group.

7. The process according to claim 6, wherein R₂ is methyl.

8. The process according to any of the claims 6-7, wherein the amine used is selected from the group consisting of allyl amine, tert-butylamine, and tert-octylamine.

9. The process according to any of the claims 6-8, wherein the amination reaction is carried out using a (C₁-C₆)-alcohol as solvent.

10. A compound of formula (IV) 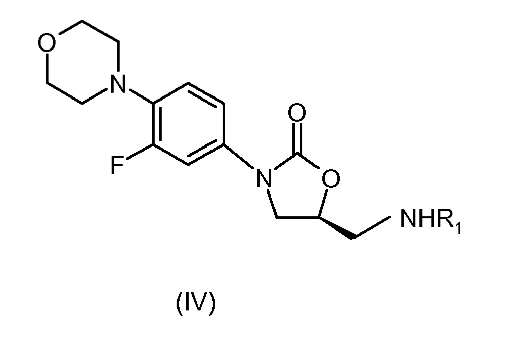 wherein R₁ is selected from the group consisting of a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group.

11. The compound according to claim 10, wherein the R₁ is selected from allyl and tert-(C₄-C₈)alkyl.

12. The compound according to claim 11, wherein R₁ is selected from the group consisting of allyl, tert-butyl, and tert-octyl.

13. A compound of formula (II) 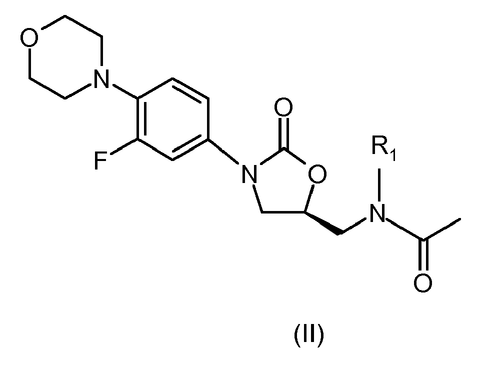 wherein R₁ is selected from the group consisting of a (C₄-C₁₀)-alkyl radical which is attached to the N atom by a tertiary C atom, and a straight or branched (C₃-C₁₀)-alkenyl radical attached to the N atom such that the C=C double bond is separated from the N atom by a methylene group.

14. The compound according to claim 13 wherein the R₁ is selected from allyl and tert-(C₄-C₈)alkyl.

15. The compound according to claim 14, wherein R₁ is selected from the group consisting of allyl, tert-butyl, and tert-octyl.
